# EUROPEAN PATENT APPLICATION

(11) **EP 2 255 794 A1**
(43) Date of publication of application: **01.12.2010**
(21) Application number: 09161601.1
(22) Date of filing: 29.05.2009
(51) Int. Cl.: A61K 9/28, A61K 9/50

(54) **Enteric coating**

(71) Applicant: H e x a l Aktiengesellschaft, 83607 Holzkirchen (DE)
(72) Inventor: Büssing, Christian, 83607 Holzkirchen (DE); Krekeler, Andreas Dr., 83607 Holzkirchen (DE); Marchesan, Marco Dr., 83607 Holzkirchen (DE)
(74) Representative: Prüfer & Partner GbR European Patent Attorneys

(57) **Abstract**

The present invention refers to an enteric coating and enteric coating composition, respectively, comprising an enteric polymer, a fatty acid and a hydrophilic plasticizer. The present invention also relates to a dosage form comprising an enteric polymer and a process for the preparation of the dosage form. Moreover, the invention refers to the use of a fatty acid for the manufacture of an enteric coating and the use of the inventive enteric coating composition for the coating of solid oral dosage forms.

## Description

### Field of the invention

The present invention belongs to the field of pharmaceutical industry and relates to an enteric coating and enteric coating compositions, respectively, comprising an enteric polymer and a plasticizer. The present invention also relates to a dosage form comprising an enteric polymer and a process for the preparation of the dosage form. The invention also refers to the use of a fatty acid for the manufacture of an enteric coating and the use of such inventive enteric coating composition for the coating of solid oral dosage forms.

### Description of the background art

Pharmaceutical dosage forms or preparations, respectively, having an enteric coating are well known in the art. The typical purpose of using such enteric coatings is protecting acid sensitive drugs from gastric acid and/or protecting the gastric mucosa from irritation or damage resulting from drugs that are not well-tolerated when released in the stomach.

When realizing such enteric coatings, usually the physical and physiological environment in the human digestive tracts has to be considered, as well as the passing time in the gastrointestinal tracts. In the gastrointestinal tracts of healthy persons, the physiological pH changes usually from acidic (pH of from about 1.8 to 4.5) to neutral (pH of from about 6.5 to 8.0) from the stomach to the small intestine and no difference is presumed to exist in the physiological environment between the small intestine and large intestine. The retention time of the preparation in the human stomach is from 0.5 to 10 hours and significantly depends on the individual person, a concurrent food intake and the size of the preparation to be administered. On the other hand, the fluctuations of a passing time through the small intestine are relatively small and the passing time is said to be 3+/-1 hours (h) (Journal of Controlled Release, 2, 27-38 (1985)).

Enteric coatings are well known in the art. For instance, the commercially available enteric coating polymers sold under the tradename EUDRAGIT^{®} (Degussa, Germany) do not dissolve in the stomach. Accordingly, EUDRAGIT^{®}S 100 dissolves at a pH value above 7.0 which corresponds to the conditions as present in the colon. This leads to the effect that the enteric coating does not dissolve in the stomach, but in the colon. A further example of an enteric coating polymer is EUDRAGIT^{®} L100-55, which dissolves at a pH value above 5.5, which means that it dissolves in the duodenum.

An enteric polymer, in admixture with a hydrophobic organic compound, is described in EP 1 101 490 A. This mixture allows further increasing the retardation of the release of the active pharmaceutical ingredient (API) compared to the use of the enteric polymer without an hydrophobic organic compound.

According to the teaching of EP 1 101 490 A, when additionally using a hydrophobic organic compound, the API is not released, even though the dosage form has reached a part of the gastrointestinal tract having conditions where the enteric polymer usually dissolves, i.e. a pH which is above a certain level. The additional period of time, wherein the API is not released, is defined as the "lag-time".

Dosage forms comprising enteric coatings are in general prepared by applying an enteric coating composition onto e.g. particles or tablets. One possible coating process for applying an enteric coating composition onto a dosage formulation is the technique of spray process. In such a process, the coating composition can e.g. be sprayed onto fluidized particles containing the API.

In order to avoid an agglomeration of API particles, which possibly occurs during spray coating, conventionally the use of glidants like e.g. talc, kaolin, glycerol monostearate and micronized SiO₂ is recommended.

Despite the above described dosage forms, there is still a need and thus an object for improved enteric coatings, enteric coating compositions and dosage forms comprising such enteric coatings.

### Summary of the invention

The present invention provides the following aspects, subject-matters and preferred embodiments, which respectively taken alone or in combination, contribute to solving the object of the present invention:
(1) An enteric coating comprising an enteric polymer or copolymer, a fatty acid and a hydrophilic plasticizer, wherein the hydrophilic plasticizer is present in the enteric coating in an amount of equal to or more than 5 wt.-% and equal to or less than 60 wt.-%, based on the amount of the enteric polymer.

In a preferred embodiment of the present invention, the enteric coating comprises a plasticizer in an amount of equal to or more than 20 wt.-% and equal to or less than 40 wt.-%, further preferred in an amount of equal to or more than 25 wt.-% and less than or equal to 35 wt.-%, based on the amount of the enteric polymer.

In a further preferred embodiment, the enteric coating comprises a plasticizer in an amount of about 30 wt.-%.
(2) The enteric coating according to item (1), wherein the enteric polymer is an anionic polymer or copolymer, preferably an anionic polymer or copolymer with methacrylic acid as a functional group.
(3) The enteric coating according to item (1) or (2), wherein the fatty acid contains at least 6 C-atoms, preferably the fatty acid comprises 6 to 22 carbon atoms, preferably 8 to 20 carbon atoms, preferably 10 to 18 carbon atoms, preferably 12 to 18 carbon atoms, further preferred the fatty acid is stearic acid.
(4) The enteric coating according to any of the preceding items, wherein the enteric coating contains the fatty acid in an amount of equal to or more than 5.5 wt.-% and equal to or less than 200 wt.-%, preferably in an amount of equal to or more than 10 wt.-% and equal to or less than 150 wt.-%, further preferred in an amount of equal to or more than 10 wt.-% and equal to or less than 100 wt.-%, still further preferred in an amount of equal to or more than 10 wt.-% and equal to or less than 50 wt.-%, particularly in an amount of equal to or more than 20 wt.-% and equal to or less than 50 wt.-%, more particularly in an amount of equal to or more than 20 wt.-% and equal to or less than 40 wt.-%, further more particularly in an amount of equal to or more than 30 wt.-% and equal to or less than 40 wt.-%, and most preferred in an amount of equal to or more than 35 wt.-% and equal to or less than 40 wt.-%, based on the amount of the enteric polymer.

In a further preferred embodiment, the enteric coating comprises fatty acid in an amount of about 40 wt.-%, based on the amount of the enteric polymer.

Within the meaning of the present invention, the term "about" denotes a deviation of +/- 10 wt.-%, preferably of +/- 5 wt.-% of the given percentages, based on the respective reference.

It is possible to prepare the enteric coating by using an enteric coating solution, preferably a solution in an organic solvent, preferably in ethanol, acetone or isopropanol, and it is also possible to prepare the enteric coating by using an enteric coating dispersion, preferably a dispersion in water. It is further suitable to use a water/solvent mixture for preparing the enteric coating.
(5) The enteric coating according to any of the preceding items, wherein the enteric coating does not have an additional retardation effect.

The term "additional retardation effect" within the meaning of the present invention denotes the retardation effect of an enteric coating comprising an enteric polymer and additives that may cause additional retardation, which substantially exceeds (e.g. by 2 hours) the retardation effect as provided by an enteric coating comprising an enteric polymer without additives that may cause additional retardation, such as fatty acids. In other words, the dosage form comprising an enteric coating as described in this specification is a delayed-release dosage form or a prolonged-release dosage form according to the European Pharmacopoeia (chapter 2.9.3., Version 01/2008: 20903). Preferably, the dosage form comprising an enteric coating is a delayed-release dosage form. A delayed-release dosage form is a dosage form which releases the active substance, e.g. the API, fractionally or totally according to the formulation design when tested in different dissolution media, e.g. in increasing pH conditions. In general, for example delayed-release gastro-resistant dosage forms require at least 2 specification points in a sequential testing and 2 different specifications in a parallel testing. In a sequential test, the first specification point is set after 1 hour (h) or 2 h in acidic medium and the second one at a pre-set time period of testing in an adequate buffer solution (preferably pH 6.8). The quantity Q, expressing the dissolution specification, has a value of 75 % after the buffer change to a pH of 6.8. This means that after the buffer change within e.g. 45 minutes (min.) 75 % of the API are dissolved or disintegrated.

Furthermore, it is also possible to determine the dissolution profile by applying a method as described below (e.g. as described in Example 5).

In a preferred embodiment, within the meaning of the present invention, the term "the enteric coating does not have an additional retardation effect" means that the enteric coating according to the present invention has a dissolution profile, wherein, when carried out an dissolution test as described below (e.g. as described in Example 5), within 5 minutes after the buffer change to pH 6.8, at least 9 %, preferably at least 12%, still preferably at least 15 %, more preferably at least 20 %, even more preferably at least 25 % and most preferably at least 30 % of the API is dissolved or disintegrated.
(6) The enteric coating as defined in any of the previous items, wherein the enteric coating contains an anionic copolymer selected from the group consisting of methacrylic acid/ethylacrylate, methacrylic acid/methyl methacrylate, and methacrylic acid/methylacrylate/methyl methacrylate.
(7) The enteric coating according to any of the preceding items, wherein the coating comprises between equal to or more than 5 wt.-% and equal to or less than 60 wt.-%, preferably between equal to or more than 20 wt.-% and equal to or less than 40 wt.-% of plasticizer, based on the amount of enteric polymer.

In a preferred embodiment, the enteric coating comprises about 30 wt.-% of plasticizer, based on the amount of enteric polymer.
(8) The enteric coating according to any of the preceding items, wherein the hydrophilic plasticizer is selected from the group consisting of phthalic derivatives, dibutylphthalate, butylphthalylbutylglycolate, propylene glycol, triacetine, silicone oil, diethylphthalate, triethyl citrate, dibutyl sebacate, polyethylene glycol and propylene glycol.
(9) A dosage form, comprising an enteric polymer as defined in any of the preceding items, wherein the dosage form preferably represents a solid oral dosage form, preferably the dosage form is a tablet, preferably a multiple unit tablet, a microtablet or a capsule which is coated with the enteric coating, or the dosage form contains crystals, granules, or pellets, which are coated with the enteric coating.

In a preferred embodiment, the solid oral dosage form is a multiple unit tablet, wherein the individual units are coated with the enteric coating.
(10) Use of a fatty acid, preferably a fatty acid as defined in item (3), for the manufacture of an enteric coating.

The use of fatty acid for the manufacture of an enteric coating can surprisingly contribute to exert various effects in combination, (i) as glidant, (ii) as separating agent, and (iii) to diminish the brittleness of the enteric coating.

The term "glidant" within the meaning of the present invention denotes an additive that prevents the agglomeration of particles containing an API, and/or that prevents the enteric coating from becoming sticky. Within this meaning, a glidant can also be an additive being denoted as a separating agent or as a lubricant.

In particular, compared to the use of conventional glidants such as talc, the use of fatty acid allows for the modulation of the mechanical properties of the enteric coating, e.g. in particular with respect to the effect of the fatty acid on increasing elasticity and diminishing brittleness. Hereby, the fatty acid, which can be considered as a "hydrophobic plasticizer", can interact with a hydrophilic plasticizer. The increased flexibility and diminished brittleness is advantageous in particular in case compression is used, where high mechanical forces are applied onto the coating, for instance in the preparation of tablets, such as multiple unit tablets.
(11) The use according to item (10), wherein the enteric coating is an enteric coating as defined in any of items (1) to (8).
(12) An enteric coating composition, comprising a fatty acid, preferably a fatty acid as defined in item (3), wherein the composition comprises between equal to or more than 5 wt.-% and equal to or less than 60 wt.-%, preferably between equal to or more than 20 wt.-% and equal to or less than 40 wt.-% of plasticizer based on the amount of enteric polymer.

It is possible that the enteric coating composition is a solution, preferably a solution in an organic solvent, preferably in ethanol, acetone or isopropanol, and it is also possible that the enteric coating composition is a dispersion, preferably a dispersion in water. It is further possible to use a water/solvent mixture for preparing the enteric coating composition.
(13) The enteric coating composition according to item (12), wherein the composition contains an anionic polymer or copolymer, preferably an anionic polymer or copolymer as defined in item (6).
(14) The enteric coating composition according to item (12) or (13), wherein the composition contains a hydrophilic plasticizer as defined in item (8).

Preferably, the hydrophilic plasticizer according to the present invention interacts with a fatty acid also being present in the enteric coating composition. The fatty acid, which can also be regarded as a hydrophobic plasticizer, interacts with the hydrophilic plasticizer, thereby e.g. increasing the flexibility and/or elasticity of the enteric coating and diminishing the brittleness of the enteric coating. Furthermore, by adding a hydrophilic plasticizer according to the present invention to an enteric coating composition, a relatively fast, yet controllable release of the API being present in the dosage form can be achieved at a desired time point, however without additional retardation time.
(15) The use of an enteric coating composition as defined in any of items (12) to (14) for the coating of solid oral dosage forms.
(16) The use according to item (15), wherein the solid oral dosage forms are solid oral dosage forms as defined in item (9).
(17) A process for the preparation of a solid oral dosage form, wherein an enteric coating composition as defined in item (13) or (14) is sprayed at least onto parts of a solid oral dosage form.

### Detailed description of the invention

The present invention is now described in more detail by preferred embodiments and examples, which are however presented for illustrative purpose only and shall not be understood as limiting the scope of the present invention in any way.

Usually, a pharmaceutical composition should fulfil certain requirements and/or should exhibit certain desired properties, which e.g. depend on the type of composition. In case the composition should not dissolve in the stomach, the pharmaceutical composition usually comprises an enteric coating.

In general, known enteric coatings may exhibit a low flexibility. Due to such a low flexibility, fissuring can occur and the damaged enteric coating film will not provide protection during stomach passage. This may lead to irritations of the stomach mucosa, or for instance to the degradation of the API being present in the pharmaceutical dosage form, as the enteric coating provides protection from the acidic environment in the stomach. Moreover, fissuring or breaking of the enteric coating may also lead to a declined bioavailability or to a reduced physiological compatibility of the API being present in the pharmaceutical composition.

Furthermore, in general, particles being present in enteric coating compositions may also tend to agglomerate, for instance during technical coating processes such as spray coating processes. The agglomeration of the particles to be coated should be avoided, as said agglomeration leads for instance to a non-homogenous size distribution of the coated particles containing the API, a nonuniform coating thickness, and the like. In order to avoid agglomeration of particles, conventional glidants such as talcum have in general been added to the enteric coating compositions. However, the addition of conventional glidants may lead to a decrease of the elasticity and/or flexibility of the resulting enteric coating. Although this effect might be reduced by the addition of conventional plasticizers, these plasticizers in turn increase a tendency of an agglomeration of the particles to be coated with the enteric coating during the coating process, e.g. the spray coating process.

In contrast thereto, the enteric coating according to the present invention is sufficiently flexible in order to avoid fissuring or breaking of the coating, for instance during the manufacturing process of the dosage form. This is in particular advantageous in case compression is used, where high mechanical forces are applied onto the coating, for instance in the preparation of tablets, such as multiple unit tablets. As a consequence, the API being present in the dosage form may exhibit an unchanged bioavailability, e.g. compared to the bioavailability of the API before compression, as well as physiological compatibility.

Furthermore, the enteric coating composition according to the present invention preferably shows a reduced agglomeration of the particles comprising the API. Therefore, e.g. deviations in the particles' size being present in the final oral dosage form such as a tablet, resulting from agglomerated particles, may be prevented. As a consequence, e.g. the dissolution properties of the coated particles may not be influenced, leading to constant dissolution profiles of the dosage forms.

In the context of the present invention, it has been unexpectedly found that the use of fatty acids and hydrophilic plasticizers in enteric coating compositions effectively avoids the formation of agglomerates in the coating process and further enhances the elasticity and flexibilty of the resulting enteric coating. Unlike conventional glidants (sometimes also referred to as separating agents or as lubricants) like talc or glycerol monostearate, the use of fatty acids as separating agents (sometimes also referred to as release agents) beneficially does not decrease the elasticity of the coating and does not lead to fissuring.

According to the instructions of the manufacturer of the EUDRAGIT^{®} polymers, in organic solutions of EUDRAGIT^{®}, the plasticizer is usually present in an amount of 10-20% based on the dry polymer substance, and in an amount of 50%-70% in aqueous dispersions (Pharma Polymere 03/2006, "Guidelines for Formulation Development and Process Technology for Enteric Coatings", 3.1e, page 2, published by Degussa, Germany).

However, it has further been unexpectedly found that by combining the use of a hydrophobic plasticizer, e.g. a fatty acid, with a hydrophilic plasticizer, e.g. a plasticizer according to the present invention, desired beneficial properties of the enteric coating regarding for instance stability, flexibility, or avoidance of agglomerations, can easiliy be adjusted over a wide concentration range of the polymers being present in the enteric coating, regardless whether an organic or a aqueous system has been chosen for the preparation of such an enteric coating.

It has further been unexpectedly found that using the above described amount of plasticizer in combination with the advantageous fatty acid in the enteric coating, an additional retardation effect may be beneficially avoided if desired, because this additional retardation is not always appropriate. Moreover, excipients such as hydrophobic organic compounds other than the aforementioned fatty acids may be beneficially avoided, thereby avoiding that such other hydrophobic compounds may provide a further retardation of the API release in addition to the retardation of the API release as already provided by the enteric polymer to be used for the enteric coating. This is particularly of advantage, as avoiding an additional lag-time or retardation time, respectively, is sometimes desired, for example if an API should be quickly released when reaching the part of the gastrointestinal tract having the desired pH level, e.g. at the beginning of the duodenum, colon or jejunum. Especially, if it is necessary to provide a therapeutic plasma level as fast as possible, the API should be directly released after passing the stomach and/or reaching the part of the gastrointestinal tract where the release is desired.

On the other hand, it is nevertheless possible to further control a possibly desired additional retardation effect brought about by the fatty acid (in addition to the retardation effect brought about by the enteric polymer itself) by adjusting the ratio of fatty acid to the plasticizer.

The enteric coatings and enteric coating compositions, respectively, according to the present invention comprise an enteric polymer, a fatty acid and a plasticizer, wherein the plasticizer is used in the specified amount.

By definition, an enteric polymer or mixture of enteric polymers does not dissolve at a pH below 5. The enteric polymer to be used according to the present invention can be one polymer or a mixture of polymers. In the following, the polymer or mixture of polymers, respectively, will be referred to as the "enteric polymer". The term "polymer" as used herein refers to homopolymers as well as to copolymers. The enteric polymer or enteric coating, respectively, does not dissolve under conditions as present in the stomach of a human or animal body. The dissolution properties of an enteric polymer or enteric coating can be determined by using e.g. in-vitro tests such as dissolution and disintegration tests. Such tests are for instance described in the European Pharmacopoeia (chapter 2.9.3.), e.g. for prolonged-release dosage forms and delayed-release dosage forms. For example with regard to delayed-type dosage forms, these in-vitro tests are usually carried out in two stages. In the first stage, the dosage form is tested in an acid phase (HCl buffer), followed by a second stage in a suitable buffer (e.g. pH 6.8, phosphate buffer). Moreover, the dissolution profile of enteric coatings can also be determined by a method as described below (e.g. as described in Example 5). Preferably the enteric polymer/enteric coating fullfils the requirements for enteric coatings as defined in the European Pharmacopoeia, chapter 2.9.3., in particular as defined for delayed-type dosage forms.

Preferably the enteric polymer is an anionic polymer, preferably at least one selected from the group consisting of enteric cellulose derivatives, enteric acrylic copolymers, enteric maleic copolymers, enteric polyvinyl derivatives and shellac. Even further preferred, the enteric polymer is an anionic polymer with methacrylic acid as a functional group. Further preferred the enteric polymer is an anionic copolymer selected from the group consisting of methacrylic acid/ethylacrylate, methacrylic acid/methyl methacrylate, and methacrylic acid/methylacrylate/methyl methacrylate.

In one embodiment, the anionic polymer or mixture of anionic polymers is used in combination with an methacrylate copolymer having neutral ester groups (e.g. R=COOCH₃ or R=COOC₄H₉) or trimethylammonioethyl groups (R=COOCH₂CH₂N+(CH₃)₃Cl) Methacrylate copolymers having a neutral ester are insoluble and pH-independent and can be used to provide a time controlled release dosage form, wherein e.g. after dissolution of the anionic polymer a matrix of the insoluble polymer remains and the medicinal substance can be washed out of this matrix.

In a preferred embodiment, the enteric polymer/enteric coating dissolves above a pH of 5.5. A dosage form comprising such an enteric coating will therefore release the API directly after leaving the stomach, e.g. in the duodenum. In another preferred embodiment of the invention, the enteric polymer/enteric coating dissolves at a pH of between 5.5 and 7.0. In another preferred embodiment, the enteric polymer/coating dissolves at a pH above 7.0. In another embodiment, the enteric polymer/enteric coating dissolves at a pH of between 5.5 and 6.5, most preferred at a pH of between 5.5 and 6.0.

Preferred enteric cellulose derivatives according to the present invention are hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose or ethylhydroxyethylcellulose phthalate.

Suitable hydroxymethylethylcellulose phthalates are also sold unter the tradename HP50^{®} and HP55^{®} (Shin-Etsu, Japan).

Preferred enteric acrylic copolymers are styrene/acrylic acid copolymer, methyl acrylate/ acrylic acid copolymer, methyl acrylate/ methacrylic acid copolymer, butyl acrylate/ styrene / acrylic acid copolymer, methacrylic acid/ methyl methacrylate copolymer, methacrylic acid/ ethylacrylate copolymer or methyl acrylate/ methacrylic acid/ octyl acrylate copolymer, or methacrylic acid/methylacrylate/methyl methacrylate.

Suitable anionic polymers or copolymers with methacrylic acid as a functional group are sold unter the tradename EUDRAGIT^{®} (Degussa, Germany): EUDRAGIT^{®} L100-55 (sold in powder form; dissolution above pH 5.5), EUDRAGIT^{®} L30 D-55 (sold as aqueous dispersion, 30%; dissolution above pH 5.5), EUDRAGIT^{®} L100 (sold in powder form; dissolution above pH 6.0) or EUDRAGIT^{®} L12,5 (sold as organic solution, 12.5%; dissolution above pH 6.0), EUDRAGIT^{®} S100 (sold in powder form; dissolution above pH 7.0), EUDRAGIT^{®} S12,5 (sold as organic solution, 12.5%; dissolution above pH 7.0) or EUDRAGIT^{®} FS 30D (sold as aqueous dispersion, 30%; dissolution above pH 7.0).

Suitable methacrylate copolymers having neutral ester groups are also sold unter the tradename EUDRAGIT^{®} (Degussa, Germany): EUDRAGIT^{®} NE 30D/ 40D /NM 30D and methacrylate copolymers having trimethylammonioethyl groups are sold unter the tradename EUDRAGIT^{®} (Degussa, Germany): EUDRAGIT^{®} RL100, EUDRAGIT^{®} RL PO/ RS PO, EUDRAGIT^{®} RL 30D/RS 30D, and EUDRAGIT^{®} RL12,5/ RS12,5.

Preferred enteric maleic copolymers are vinylacetate/ maleic acid anhydride copolymer, styrene/ maleic acid anhydride copolymer, styrene/ maleic acid monoester copolymer, vinylmethylether / maleic acid anhydride copolymer, ethylene/ maleic acid anhydride copolymer, vinylbutylether/ maleic acid anhydride copolymer, acrylonitrile/ methyl acrylate/ maleic acid anhydride copolymer or butyl acrylate/ styrene/ maleic acid anhydride copolymer.

Preferred enteric polyvinyl derivatives are polyvinyl alcohol phthalate, polyvinylacetal phthalate, polyvinyl butylate phthalate or polyvinylacetoacetal phthalate.

Preferably the amount of enteric polymer in the enteric coating is between equal to or more than 30 wt.-% and equal to or less than 85 wt.-%, further preferred between equal to or more than 30 wt.-% and equal to or less than 80 wt.-%, further preferred between equal to or more than 30 wt.-% and equal to or less than 75 wt.-%, even further preferred between equal to or more than 30 wt.-% and equal to or less than 70 wt.-%, further preferred between equal to or more than 30 wt.-% and equal to or less than 65 wt.-%, and further preferred between equal to or more than 30 wt.-% and equal to or less than 60 wt.-%, even further preferred between equal to or more than 30 wt.-% and equal to or less than 50 wt-%, and most between equal to or more than 30 wt.-% and equal to or less than 40 wt.-%, based on the amount of the enteric coating.

As a constituent of the enteric coating according to the present invention, any fatty acid can be used. Preferably such a fatty acid has at least 6 carbon atoms, further preferred between 6 and 22 carbon atoms. Particularly preferred, such a fatty acid comprises 8 to 20 carbon atoms, still particularly preferred 10 to 18 carbons and especially preferred 12 to 18 carbon atoms. The fatty acid can be saturated or unsaturated, preferably the fatty acid is a saturated acid. Preferably the saturated fatty acid is selected from the group consisting of stearic acid, lauric acid, myristic acid, palmitic acid or behenic acid, particular preferred, stearic acid is used. According to the present invention, the above-mentioned fatty acids may be employed not only alone but also in a mixture of two or more thereof.

Preferably the amount of fatty acid to be used according to the invention is in an amount of equal to or more than 5.5 wt.-% and equal to or less than 200 wt.-%, preferably in an amount of equal to or more than 10 wt.-% and equal to or less than 150 wt.-%, further preferred in an amount of equal to or more than 10 wt.-% and equal to or less than 100 wt.-%, still further preferred in an amount of equal to or more than 10 wt.-% and equal to or less than 50 wt.-%, particularly in an amount of equal to or more than 20 wt.-% and equal to or less than 50 wt.-%, more particularly in an amount of equal to or more than 20 wt.-% and equal to or less than 40 wt.-%, further preferred in an amount of equal to or less than 30 wt.-% and equal to or less than 40 wt.-%, and most preferred in an amount of equal to or less than 35 wt.-% and equal to or less than 40 wt.-%, based on the amount of the enteric polymer.

It is possible to prepare the enteric coating by using an enteric coating solution, preferably a solution in an organic solvent, preferably in ethanol, acetone or isopropanol, and it is also possible to prepare the enteric coating by using an enteric coating dispersion, preferably a dispersion in water. It is further possible to use a water/solvent mixture for preparing the enteric coating.

The plasticizer which can be used according to the present invention can be a pharmaceutically acceptable plasticizier which can be used in combination with an enteric polymer to provide an enteric coating. In general, plasticizers are low-molecular substances that should exhibit a dissolution profile similar to that of the enteric polymer. Furthermore, they should be homogenously distributable in the enteric polymer. The specific interaction between the plasticizer and the enteric polymer, the plasticizers among each other and the enteric polymers among each other leads to an increased flexibility of the enteric polymer, which is also reflected in a change of physical properties of the enteric polymer. For instance, the glas transition temperature and brittleness decrease with increasing flexibility.

Plasticizers are known in the art and may, in principle, be hydrophilic or hydrophobic molecules. Within the meaning of the present invention, the plasticizers are preferably hydrophilic plasticizers. In a preferred embodiment, the hydrophilic plasticizers according to the present invention interact with a fatty acid also being present in the enteric coating composition or in the enteric coating, respectively. The fatty acid, which can also be regarded as a hydrophobic plasticizer, interacts with the hydrophilic plasticizer, thereby e.g. increasing the flexibility and/or elasticity of the enteric coating and diminishing the brittleness of the enteric coating. Furthermore, by adding a hydrophilic plasticizer according to the present invention to an enteric coating composition, a relatively fast, yet controllable release of the API being present in the dosage form can be achieved.

Further preferred, the plasticizers are selected from the group consisting of phthalic derivatives, dibutylphthalate, butylphthalylbutylglycolate, propylene glycol, triacetine, silicone oil, diethylphthalate, triethyl citrate, dibutyl sebacate, polyethylene glycol and propylene glycol, further preferred, the plasticizer is selected from triethyl citrate, dibutyl sebacate, polyethylene glycol and propylene glycol. More preferably triethyl citrate and propylene glycol is used, most preferably propylene glycol.

According to the present invention, the plasticizer is used in an amount of equal to or more than 5 wt.-% and equal to or less than 60 wt.-%, further preferred in an amount of equal to or more than 20 wt.-% and less than or equal to 40 wt.-%, and even further preferred in an amount of equal to or more than 25 wt.-% and equal to or less than 35 wt.-%, based on the amount of the enteric polymer. In a preferred embodiment, the plasticizer is used in an amount of about 30 wt.-% based on the amount of enteric polymer.

The combination of the described amount of plasticizer with the fatty acid leads to an enhanced elasticity and/or flexibility of the enteric coating. This is in particular advantageous in case enteric coated particles are formulated into dosage forms by using a compression process, where high pressures are applied onto the coated particles. This is for instance the case if the enteric coated particles are compressed into tablets, in particular in multiple unit tablets. In case the flexibility of the coating is too low, fissuring can occur. The damaged film is not able to protect the API during the passage through the stomach and/or to protect the stomach from the potential irritating effects of the API.

It is additionally preferred to use the plasticizer and fatty acid in amounts that avoid an additional retardation effect of the fatty acid. A suitable test for determining whether a dosage form possesses an additional retardation effect is the disintegration test as described below (Example 5). In brief, the API, together with pharmaceutical acceptable excipients, has been applied onto non-pareilles (sugar cores). These API loaded non-pareilles in turn have been coated with various coatings containing Eudragit L100-55, a plasticizer (e.g. propylene glycol) and stearic acid. Afterwards, the dissolution profiles of the obtained pellets have been determined by HPLC measurement. For this purpose, the pellets containing the different enteric coatings have been pre-treated for 2 hours in 1000 ml 0.05 M sodium acetate (pH 4.5), followed by filtering off of the pellets. The pellets were then transferred to 900 ml 0.1 Na₂HPO₄ (pH 6.8), where the solution containing the pellets has been stirred with a paddle speed of 100 rpm (rotations per minute). The released API (such as esomeprazole) in % has been detected at different time points (0, 5, 15, 30, 45 minutes (min.)) at a wavelength of 303 nm.

It is additionally preferred to include a coloring agent in the enteric coating and enteric coating composition, respectively. Examples of the coloring agent include for instance food dyes, lake dyes, caramel, carotenes, annatto, cochineal, iron dioxides and the like, and an opaque coloring agent, OPALUX, mostly made of a lake dye and syrup. Examples of food dyes include food aluminum lakes such as food red No.2, No.3, yellow No.4, No. 5, green No. 3, blue No. 1, No. 2 and purple No.1; annatto (natural color derived from Bixa orellana); carmine (aluminum carminate); pearl essence which mainly consists of guanine, and the like.

It is additionally preferred to include a masking agent in the enteric coating and enteric coating composition, respectively. Examples of a masking agent include titanium dioxide, precipitated calcium carbonate, dibasic calcium phosphate, calcium sulfate, and the like.

It is additionally preferred to include a lubricant in the enteric coating and enteric coating composition, respectively. Examples of the lubricant encompass magnesium stearate, talc, synthetic magnesium silicate, fine grained silicon oxide, and the like.

In addition, other excipients can be included in the enteric coating according to the present invention. Such excipients are also described below. It is additionally preferred to include surfactants, preferably non ionic surfactants like e.g. polysorbates, e.g. polysorbate 80 or 20, into the enteric coating.

As described in more detail below, a dosage form comprising an enteric coating can be produced by coating a subject with an enteric coating composition which is an enteric coating solution or dispersion of the components.

It has been unexpectedly found that enteric coatings having particularly good physical properties for instance with regard to elasticity, flexibility and inhibition of agglomeration during the coating process, can be provided, if the amount of plasticizer and fatty acid is specifically chosen depending on the type of enteric coating composition to be used (e.g. enteric coating compositions as a solution or dispersion). Preferably organic solvents like e.g. ethanol, acetone, isopropanol, and the like are used to prepare enteric coating solutions.

Preferably the enteric coating compositions comprise between equal to or more than 5 wt.-% and equal to or less than 60 wt.-% of plasticizer, further preferred the amount of plasticizer is between equal to or more than 20 wt.-% and equal to or less than 40 wt.-%, even further preferred between equal to or more than 25 wt.-% and equal to or less than 35 wt.-%, based on the amount of enteric polymer.

When preparing enteric coating compositions as dispersions, preferably water is used as the solvent. Furthermore, it is preferred to use an amount of plasticizer which is between equal to or more than 5 wt.-% and equal to or less than 60 wt.-%, further preferred between equal to or more than 20 wt.-% and equal to or less than 40 wt.-%, and most preferred between 25 wt.-% and equal to or less than 35 wt.-%, based on the amount of enteric polymer. Whether an enteric coating has been prepared by using an enteric coating solution or by using an enteric coating dispersion, can for instance be determined by detecting residues of the solvent used (e.g. the solvents as described herein, such as water, or acetone, isopropanol, ethanol) being present in or on the enteric coating, or by visual inspection of the enteric coating with the help of a scanning electron microscope (SEM). A person skilled in the art is able to assess via an SEM-analysis, whether an enteric coating has been prepared by using a coating solution or a coating dispersion. For instance, in case a solution has been used, the enteric coating is more compact compared to an enteric coating that has been prepared by using a coating solution.

The present invention also refers to a pharmaceutical dosage form which comprises an enteric coating as described herein. Such (pharmaceutical) dosage forms can be any dosage forms which can contain an enteric coating. The enteric coating may represent the outer layer of such a dosage form, or may be a layer which is beneath other layers or forms a layer of a part of the tablet. For example, the dosage form may be a capsule or a tablet, preferably a tablet, which comprises an enteric coating as an outer layer, or the capsule or tablet may also be soluble in the stomach but contains multiple units like granules containing the API, wherein the granules contain an enteric coating as an outer layer. In a preferred embodiment, the dosage form is a multiple unit tablet. The dosage form can also be a tablet which is coated with an enteric coating. In another embodiment, the tablet comprises an outer layer which alleviates the swallowing of the tablet, wherein the enteric coating is beneath the outer layer. If desired, a further overcoating may be applied on top of the enteric coating. Such overcoatings are well known in the prior art.

Preferably the dosage form is a solid oral dosage form, further preferred the dosage form is a tablet, such as a multiple unit tablet, microtablet (e.g. tablets having a diameter of less than 1 mm) or a capsule, which is coated with the enteric coating. It may also be possible that the dosage form contains crystals, granules, spheroids or pellets, which are coated with the enteric coating. A spheroid which is enterically coated can e.g. be a non-pareille.

The enteric coating according to the present invention is used in amounts which are suitable to provide the desired dissolution profile and which inhibit the release of the API during its passage through the stomach. After leaving the stomach, the API can be quickly released or can be released in a sustained manner. In a preferred embodiment of the invention, after leaving the stomach, releasing of the API preferaby begins without essential additional retardation, e.g. without an additional lag time or retardation time, respectively, as defined above.

It is additionally preferred to use an amount of coating in a range of from 5 wt.-% to 300 wt.-%, further preferred in a range of from 20 wt.-% to 100 wt.-% based on the total amount of ingredients of the dosage form which are not part of the enteric coating.

The enteric coating according to the present invention can be prepared by using any suitable method which is known in pharmaceutical technology for preparing enteric coatings. Usually, an enteric coating is prepared by applying an enteric coating composition onto the subject to be coated (core material), e.g. a particular material or a tablet or a capsule.

The present invention therefore also refers to an enteric coating composition which can be used to apply the enteric coating onto the desired subjects. The enteric coating composition comprises at least the enteric polymer, the fatty acid, the plasticizer and a solvent. In general, the enteric coating corresponds to the enteric coating composition without the solvent of the enteric coating composition. Therefore, all preferred embodiments and preferred components and amounts of components as described for the enteric coating can also be used in the enteric coating composition, which additionally comprises a solvent in order to provide a solution or dispersion of the components of the enteric coating.

Preferably the coating composition contains a suitable solvent. The enteric coating composition can e.g. be prepared by using the information as provided by the supplier of the enteric polymer. For example, aqueous dispersions of EUDRAGIT^{®} polymers can be prepared by stirring EUDRAGIT^{®} powder in water and adding alkali in order to partially neutralize the carboxylic acid groups of the polymer, resulting in the formation of a colloidal dispersion.

For example, enteric coatings can be prepared by applying 4 to 6 mg of enteric polymer per cm² of tablet surface, or 10 wt.-% to 30 wt.-% on particles such as pellets, granules or crystals with sizes in the range of about 0.5 to 1.2 mm.

Depending on the type of enteric polymer, the fatty acid, the plasticizer, the optional further excipients and the solvent, two types of enteric coating compositions exist, namely solutions and dispersions. According to the invention, the enteric coating composition can contain one or more than one solvent, preferably only one solvent is used.

The enteric polymer is usually soluble in organic solvents like ethanol, isopropanol or acetone and the enteric coating compositions can be provided as a solution. When using water as the solvent, the enteric polymer does not dissolve and the enteric coating composition is provided as dispersion. However, it is possible that some of the components of the enteric coating composition are not dissolved due to their low solubility in the solvent or the high amount in which they are present in the enteric coating composition. Preferably the amount of dry substance in the solution or dispersion is between 10 wt.-% and 40 wt.-%, preferably between 20 wt.-% and 35 wt.-%, and more preferred between 20 wt.-% and 30 wt.-%, based on the amount of the solution or dispersion.

Suitable amounts of solvents can be chosen by a person skilled in the art. Preferably the amounts of solvents are chosen in order to provide a solution or dispersion of components which is most suitable for the technical coating process, e.g. a spray coating process. In case an organic solvent is used to prepare an enteric coating solution, the solvent is preferably used at least in an amount which is necessary to dissolve all the components of the enteric coating composition. In a preferred embodiment of the invention, the overall concentration of components in the solution is between 10 wt.-% and 30 wt-%, further preferred between 10 wt.-% and 20 wt.-%. The solvent to be used for the enteric coating solution according to the invention is preferably selected from the group consisting of: alcohols such as methanol, ethanol, n-propanol, isopropanol, n-butanol, 2-methoxyethanol and 2-ethoxyethanol; hydrocarbons such as hexane, cyclohexane, petroleum ether, petroleum benzine, ligroin, benzene, toluene and xylene; ketones such as acetone and methylethylketone; hydrocarbon halides such as dichloromethane, chloroform, carbon tetrachloride, ethylene dichloride, trichloroethylene and 1,1,1-trichloroethane; esters such as methyl acetate, ethyl acetate and butyl acetate; ethers such as isopropyl ether and dioxane. Preferably the solvent to be used is an alcohol, preferably ethanol is used. According to the invention, one type of solvent or a mixture of solvent can be used.

The method for preparing the preparation, e.g. the solid oral dosage form, of the present invention is not limited as long as the dosage form contains an enteric coating which avoids the releasing of the API in the stomach. The dosage form is preferably obtained by using a spray-coating process, wherein a core material containing an API is coated with an enteric coating composition according to the invention.

The spray coating process can be carried out with any known coating machine. Examples of the coating machine include a fluidized bed coating machine, a centrifugal fluidized bed coating machine, a pan coating machine and the like. The core particle can consist of the API, or can be a mixture of one or more APIs with various excipients. Examples of the core particle that may be coated include tablets, capsules and the like, as well as particles such as grains, crystals, pellets, spheroids and granules.

Suitable temperatures that may be used for the coating process are well known to a person skilled in the art. For example, a fluid bed coating process using an aqueous enteric coating dispersion can be carried out at 20-30°C. The coating process in a pan coater can be carried out at 30-35°C. When using organic solutions, about 25°C are suitable for the fluid bed process and about 30°C for the pan coater process.

The enteric coating composition is applied onto the particular material, tablets or capsules as described above. The material to be coated (core material) according to the invention can comprise the API or a mixture of APIs and optional one or more excipients.

In the dosage form according to the invention, any API which is suitable for oral administration can be used. Examples of various medicinal substances (APIs) include, without being limited to, antifungal agents like e.g. fluconazol; non steroidal anti-inflammatory drugs, such as antipyretic analgesic anti-inflammatory agents (for example, indomethacin, aspirin, diclofenac sodium, ketoprofen, ibuprofen, mefenamic acid, azulene, phenacetin, isopropylantipyrine, acetaminophen, bendzac, phenylbutazone, flufenamic acid, sodium salicylate, salicylamide, sasapyrine, etodolac and the like); proton pump inhibitors (for example, prazoles like lansoprazole, omeprazole, esomeprazole, pantoprazole, rabeprazole, leminoprazole, and the like); steroidal anti-inflammatory agents (for example, dexamethasone, hydrocortisone, prednisolone, triamcinolone and the like); antiulcer agents (for example, 5-amino salicylic acid, ecabet sodium, enprostil, sulpiride, cetraxate hydrochloride, gefarnate, irsogladine maleate, cimetidine, ranitidine hydrochloride, famotidine, nizatidine, roxatidine acetate hydrochloride and the like); coronary vasodilators (for example, nifedipine, isosorbide dinitrate, diltiazem hydrochloride, trapidil, dipyridamole, dilazep dihydrochloride, verapamil, nicardipine, nicardipine hydrochloride, verapamil hydrochloride and the like); peripheral vasodilators (for example, ifenprodil tartrate, cinepazet maleate, cyclandelate, cinnarizine, pentoxifylline and the like); antibiotics (for example, ampicillin, amoxicillin, cephalexin, erythromycin ethylsuccinate, bacampicillin hydrochloride, minocycline hydrochloride, chloramphenicol, tetracycline, erythromycin, ceftazidime, cefuroxime sodium, aspoxicillin, ritipenem acoxil hydrate and the like); synthetic antimicrobials (for example, nalidixic acid, piromidic acid, pipemidic acid trihydrate, enoxacin, cinoxacin, ofloxacin, norfloxacin, ciprofloxacin hydrocloride, sulfamethoxazole trimethoprim and the like); antiviral agents (for example, aciclovir, ganciclovir and the like); antispasmodics (for example, propantheline bromide, atropine sulfate, oxapium bromide, timepidium bromide, butylscopolamine bromide, trospium chloride, butropium bromide, N-methyl scopolamine methyl sulfate, methyloctatropine bromide and the like); antitussives (for example, tipepidine hibenzoate, methylephedrine hydrochloride, codeine phosphate, tranilast, dextromethorphan hydrobromide, dimemorfan phosphate, clobutinol hydrochloride, fominoben hydrochloride, benproperine phosphate, eprazinone hydrochloride, chlophedianol hydrochloride, ephedrine hydrochloride, noscapine, pentoxyverine citrate, oxeladine citrate, isoaminil citrate and the like); expectorants (for example, bromhexine hydrochloride, carbocysteine, ethylcysteine hydrochloride, methylcysteine hydrochloride and the like); bronchodilators (for example, theopylline, aminophylline, disodium cromoglycate, procaterol hydrochloride, trimetoquinol hydrochloride, diprophylline, salbutamol sulfate, clorprenaline hydrochloride, formoterol fumarate, orciprenalin sulfate, pirbuterol hydrochloride, hexoprenaline sulfate, bitolterol mesilate, clenbuterol hydrochloride, terbutaline sulfate, mabuterol hydrochloride, fenoterol hydrobromide, methoxy phenamine hydrochloride and the like); cardiacs (for example, dopamine hydrochloride, dobutamine hydrochloride, docarpamine, denopamine, caffeine, digoxin, digitoxin, ubidecarenon and the like); diuretics (for example, furusemide, acetazolamide, trichlormethiazide, methyclothiazide, hydrochlorothiazide, hydroflumethiazide, ethiazide, cyclopenthiazide, spironolactone, trimterene, fluorothiazide, piretanide, mefruside, ethacrynic acid, azosemido, clofenamide and the like); muscle relaxants (for example, chlorphenesin carbamate, tolperisone hydrochloride, eperisone hydrochloride, tizanidine hydrochloride, mephenesin, chlorzoxazone, phenprobamate, methocarbamol, chlormezanone, pridinol mesylate, afloqualone, baclofen, dantrolene sodium and the like); brain metabolism improvers (for example, nicergoline, meclofenoxate hydrochloride, taltirelin and the like); minor tranquilizers (for example, oxazolam, diazepam, clotiazepam, medazepam, temazepam, fludiazepam, meprobamate, nitrazepam, chlordiazepoxide and the like); major tranquilizers (for example, sulpiride, clocapramin hydrochloride, zotepine, chlorpromazine, haloperidol and the like); beta -blockers (for example, bisoprolol fumarate, pindolol, propranolol hydrochloride, carteolol hydrochloride, metoprolol tartrate, labetalol hydrochloride, acebutolol hydrochloride, bufetolol hydrochloride, alprenolol hydrochloride, arotinolol hydrochloride, oxprenolol hydrochloride, nadolol, bucumolol hydrochloride, indenolol hydrochloride, timolol maleate, befunolol hydrochloride, bupranolol hydrochloride and the like); antiarrhythmic agents (for example, procainamide hydrochloride, disopyramide, ajmaline, quinidine sulfate, aprindine hydrochloride, propaphenone hydrochloride, mexiletine hydrochloride, azimilide hydrochloride and the like); antipodagrics (for example, allopurinol, probenecid, colchicine, sulfinpyrazone, benzbromarone, bucolome and the like); anticoagulants (for example, ticlopidine hydrochloride, dicumarol, warfarin potassium, (2R,3R)-3-acetoxy-5-[2-(dimethylamino)ethyl]-2,3-dihydro-8-methyl-2-(4-methylphenyl)-1,5-benzothiazepine-4(5H)-on · maleate and the like); thrombolytic agents (for example, methyl(2E,3Z)-3-benzyliden-4-(3,5-dimethoxy- alpha - methylbenzyliden)-N-(4-methylpiperazine-1-yl)succinamate · hydrochloride and the like); agents for liver disease (for example, (+/-)r-5-hydroxymethyl-t-7-(3,4-dimethoxyphenyl)-4-oxo-4,5,6,7-tetrahydrobenzo[b]furan-c-6-carbonate lactone and the like); antiepileptics (for example, phenytoin, sodium valproate, metharbital, carbamazepine and the like); antihistamines (for example, chlorpheniramine maleate, clemastine fumarate, mequitazine, alimemazine tartrate, cycloheptazine hydrochloride, bepotastine besilate and the like); antiemetics (for example, difenidol hydrochloride, metoclopramide, donperidone, betahistine mesylate, trimebutine maleate and the like); hypotensive agents (for example, dimethylaminoethylreserpinate dihydrochloride, recinnamine, methyldopa, prazosin hydrochloride, bunazosin hydrochloride, clonidine hydrochloride, budralazine, urapidil, N-[6-[2-[(5-bromo-2-pyrimidinyl)oxy]ethoxy]-5-(4-methylphenyl)-4-pyrimidinyl]-4-(2-hydroxy-1,1-dimethylethyl)benzenesulfonamide sodiumsalt and the like); agents for hyperlipemia (for example, pravastatin sodium, fluvastatin sodium and the like); sympathomimetic agents (for example, dihydroergotamine mesylate, isoproterenol hydrochloride, etilefrine hydrochloride and the like); oral antidiabetics (for example, glibenclamide, tolbutamide, glymidine sodium and the like); oral carcinostatic agents (for example, marimastat and the like); alkaloid narcotic (for example, morphine, codeine, cocaine and the like); vitamins (for example, vitamin B1, vitamin B2, vitamin B6, vitamin B12, vitamin C, folic acid and the like); agents for treatment of pollakisuria (for example, flavoxate hydrochloride, oxybutynin hydrochloride, terodiline hydrochloride and the like); angiotensin converting enzyme inhibitors (for example, imidapril hydrochloride, enalapril maleate, alacepril, delapril hydrochloride and the like); and the like.

The selected API(s) may be present as enantiomer, e.g. as (+)- or (-)-enantiomer (such as, e.g., esomeprazole), or as racemate. Furthermore, it is possible that only one API is present in a solid oral dosage form, and it is also possible that a combination of APIs is present in a solid oral dosage form. Therefore, the API, whether it is the same API or a combination of different APIs, may be present in different units or compartments of the dosage form, in particular of the multiple unit dosage form, for instance in the units being present in a multiple unit tablet. Furthermore, it is also possible that the API is present in the core and in the coating of a solid oral dosage form.

In a preferred embodiment, the API(s) is selected from the group consisting of antifungal agents (e.g. fluconazol), non-steroidal anti-inflammatory drugs, proton pump inhibitors such as the prazoles, and antibiotics. Among these are APIs which are particularly sensitive with regard to an acidic pH as being present in the stomach. Therefore, such API(s) can be effectively protected by the enteric coating according to the present invention as disclosed herein from being degraded in the stomach and/or from causing irritations of the stomach mucosa.

Examples of the excipients include, for instance, vehicles such as lactose, saccharose, mannitol, xylitol, erythritol, sorbitol, maltitol, calcium citrate, calcium phosphate and microcrystalline cellulose; disintegrants such as corn starch, potato starch, sodium carboxymethyl starch, partly pregelatinized starch, carboxymethylcellulose calcium, carboxymethylcellulose, low substituted hydroxypropylcellulose, crosslinked carboxymethylcellulose sodium and crosslinked polyvinylpyrrolidone; binders such as hydroxypropylcellulose, hydroxypropylmethylcellulose, polyvinylpyrrolidone, polyethyleneglycol, dextrin and pregelatinized starch; lubricants such as magnesium stearate, calcium stearate, talc, light anhydrous silicic acid and hydrated silicon dioxide; further, surface active agents such as phospholipid, glycerin ester of fatty acid, sorbitan ester of fatty acid, polyoxyethylene ester of fatty acid, polyethyleneglycol ester of fatty acid, polyoxyethlene hydrogenated castor oil, polyoxyethlene alkylether and sucrose ester of fatty acid; furthermore, aromatics such as orange and strawberry, coloring matters such as sesqui-iron oxide, yellow sesqui-iron oxide, Food yellow No.5, Food yellow No.4 and aluminum chelate; sweetening agents such as saccharin and aspartame; corrigents such as citric acid, sodium citrate, succinic acid, tartaric acid, fumaric acid and glutamic acid; solubilizing agents such as cyclodextrin, arginine, lysine and trisaminomethane.

When grains or granules are employed as a core particle, they can be prepared by known methods for granulation such as wet granulation, dry granulation, layering granulation and impregnate granulation.

The core material which is coated with the enteric coating composition can be manufactured by using any known method.

In wet granulation, according to a conventional method, granulation and/or grading may be carried out by granulating with an agitating granulator, a high speed mixer granulator and the like after a binder solution has been added to a mixture in which an API is mixed with various additives for the preparation, or by employing an oscillating granulating machine after kneading and adding a binding solution to a mixture of an API and various additives for the preparation. Further, granulation can be carried out by employing a fluidized bed granulator, agitation fluidized bed granulator and the like with spraying a binder solution to a fluidizing mixture of an API and various additives for preparations.

In dry granulation, a mixture of an API and various additives may be granulated by employing a roller compactor, a roll granulator and the like.

In case a layering granulation is carried out, an API (if necessary, together with other pharmaceutical additives) is added to a rolled inert carrier, while a binder solution which has to be attached onto the carrier is sprayed using a centrifugal fluidized bed coater and the like.

Examples of the inert carrier include, for instance, crystallines of sugars or inorganic salts such as crystalline of lactose, microcrystalline cellulose, crystalline of sodium chloride, a spherical granulated material such as the spherical granulated material of crystalline cellulose (available from Asahi Chemical Industry Co., Ltd.; Trade-name: Avicel SP), the spherical granulated material of crystalline cellulose and lactose (available from Freund Industrial Co., Ltd.; Trade-name: Nonpareil NP-5, and Nonpareil NP-7), the spherical granulated material of refined sugar (available from Freund Industrial Co., Ltd.; Trade-name: Nonpareil-103), and the spherical granulated material of lactose and alpha -starch.

In impregnating granulation, usually suitable concentrations of API solution are mixed with a porous carrier in order to keep the API solution into the pore portion of the carrier, and then the mixture may be dried in order to remove the solvent.

Examples of a porous carrier include, for instance, aluminum magnesium metasilicate (available from Fuji Chemical Industry Co., Ltd.; Trade-name: NEUSILIN), calcium silicate (available from Eisai Co., Ltd.; Trade-name: FLORITE) and the like.

When a tablet is used as a core material, a mixture of an API and various pharmaceutical additives is press-formed as such, or excipients like a disintegrator, a lubricant or the like are added to the mixture after having formed a granulated product with the above-mentioned method into the form of a tablet.

When a capsule is used as a core material, a hard capsule or soft capsule, which is filled with an API, may be used.

The dosage form according to the present invention represents a solid oral dosage form, preferably the dosage form is a tablet, microtablet (e.g.smaller than 1 mm) or capsule, which is coated with the enteric coating, or the dosage form contains crystals, granules, or pellets, which are coated with the enteric coating according to the present invention.

In a further aspect, the present invention relates to the enteric coating composition according to the present invention, wherein the composition contains an anionic polymer or copolymer as described herein.

Furthermore, the present invention relates to the use of an enteric coating composition according to the present invention for the coating of solid oral dosage forms.

In a further aspect, the present invention is directed to a process for the preparation of a solid oral dosage form, wherein an enteric coating composition as defined herein is sprayed at least onto parts of a solid oral dosage form.

The following drawings and examples illustrate the present invention in more detail, which are however presented for illustrative purpose only and shall not be understood as limiting the scope of the present invention in any way.

### Examples

### Example 1: Preparation of enteric coating composition

### Composition 1:

4500g water are filled into a 12-15 1 vessel and 3000g EUDRAGIT L 100-55 are added in portions with stirring. By doing so, it is ensured that the powder is rapidly wetted and dispersed without lump and foam formulation. After stirring for 5 to 10 minutes, 1000g 1N NaOH are added in a thin stream to the top the vortex within 5 minutes, and stirring is then continued for another 30 minutes. Under stirring, 600g triethyl citrate are added.

### Composition 2:

1500g water are filled into a 5 1 vessel and 600g stearic acid are added in portions with homogenizing via ultraturrax for 15 - 30min.

During low stirring, composition 2 is added into composition 1.

### Example 2: Spray coating of granules

The granules to be coated are filled into the coater and are fluidized by adjusting the air supply. The coating composition is sprayed onto the the fluidized particles, which are prewarmed to about 30°C, by means of an air borne spray gun. Spray rate, inlet air quantity and inlet air temperature are adjusted in such a way that spraying can be performed continuously without the formation of agglomerates. During the process, the crystals or granules should be maintained at a temperature between 25 and 30°C and be able to flow freely.

### Example 3: Flexibility of inventive and comparison coatings

For determining the flexibility of the inventive (formulation 1) and the prior art (comparison formulations 2 to 7) enteric coatings have been subjected to a flexibility test as described in DIN EN 14477.

### 1. Preparation of the enteric coatings

In brief, the apparatus used for the preparation of the respective enteric coatings was a Erichsen Coatmaster 509/MC-I. The foil, on which the enteric coating to be tested is prepared, was a PET foil 74/450. This foil was cut out and fixed onto the Coatmaster on the designated area, the coat thickness of 500 µm was adjusted with the help of the adjusting screw, and the prepared enteric coating composition has been poured onto the foil. Then, the enteric coating composition has been stretched until a coat thickness of 500 µm has been reached. The film has been dried on air for several minutes, and afterwards put into a drying chamber for about 1 h at 45°C. Finally, the film has been kept in a desiccator with orange gel for about 12 h.

### 2. Testing of the enteric coatings

The speed for testing was chosen to be 5mm/min (endpoint of measurement was chosen to be 1 min of process duration). The enteric coatings have been prepared as indicated above by using the amounts of components as given in table I. However, it has to be noted that (for comparability reasons) the solid substance concentration (mg/surface) is chosen such that the thickness of the enteric coatings to be tested is essentially the same, for example 500 µm. The data referring to the maximum force and maximum elongation was given in table 1 show that formulation 1, representative for the present invention, displayed the best flexibility and the enteric coating did not break under the testing conditions.

**Table I:**

| **Component** | **Formulation 1** | **Comparison Formulation 2** | **Comparison Formulation 3** | **Comparison Formulation 4** | **Comparison Formulation 5** | **Comparison Formulation 6** | **Comparison Formulation 7** |
|---|---|---|---|---|---|---|---|
| Eudragit L100-55 (enteric polymer) | 14.29 g 100 parts by weight | 11.76 g 100 parts by weight | 16.67 g 100 parts by weight | 13.33 g 100 parts by weight | 14.29 g 100 parts by weight | 10.00 g 100 parts by weight | 20.00 g 100 parts by weight |
| Triethyl citrate (plastic i-zer) | 2.86 g 20 wt.- % * | 2.35 g 20 wt.- % * | 3.33 g 20 20 wt.-% * | | | | |
| Stearic acid (fatty acid) | 2.86 g 20 wt.- % * | | | 6.67 g 50 wt.- % * | 5.71 g 40 wt.- % * | 10.00 g 100 wt.-% * | |
| Talc | | 5.88 g 50 wt-% * | | | | | |
| Concen-tration | 10 wt.- % | 10 wt.- % | 10 wt.- % | 10 wt.- % | 10 wt.- % | 10 wt.- % | 10 wt.-% |
| of enteric coating composi-tion | (in ethanol ) | (in ethanol | (in ethanol | (in ethanol | (in ethanol | (in ethanol | (in ethanol |
| Ethanol (solvent ) | 180 g | 180 g | 180 g | 180 g | 180 g | 180 g | 180 g |
| Fₘₐₓ (maximum force) | 2.22 N; 4.90 mm elonga-tion **(no breaking of coating )** | 3.91 N; 0.75 mm elongat ion (coatin g was broken) | 9.36 N; 3.40 mm (coatin g was broken) | 0.64 N; 0.43 mm (coatin g was broken) | 1.50 N; 0.81 mm (coatin g was broken) | 0.49 N; 0.38 mm (coatin g was broken) | 12,14 N 1.16 mm (coatin g was broken) |

| | | | | | | | |
|---|---|---|---|---|---|---|---|
| *: Based on the amount of the enteric polymer (Eudragit L100-55) | | | | | | | |

### Example 4:

Moreover, a further enteric coating according to the present invention has been prepared, wherein the enteric coating composition has been prepared as described in example 1.

| | | | | |
|---|---|---|---|---|
| Eudragit L 100-55: | | 305.24 g | | 100 parts by weight |
| Triethyl citrate: | | 91.57 g | 30 wt.-% * | |
| Stearic acid: | 122.09 g | 40 wt.-% * | | |
| Tween 80: | 6.10 g | 2 wt.-% * | | |
| Ethanol: | 1575.00 g | | | |

| | | | | |
|---|---|---|---|---|
| *: Based on the amount of the enteric polymer (Eudragit L100-55) | | | | |

In this example, the API used was acetylsalicylic acid (ASS).

### Example 5: Dissolution profiles

### 1. Influence of stearic acid on the dissolution profile

In order to show the influence of stearic acid on the dissolution profile of the enteric polymer, the dissolution of an API (esomeprazole) coated with the respective enteric coating has been examined (Dissolution appareil: e.g. Sotax AT7). For this purpose, the API, together with pharmaceutical acceptable excipients, has been applied onto non-pareilles (sugar cores). These non-pareilles in turn have been coated with various coatings containing Eudragit L100-55 and a plasticizer (propylene glycol) in constant amounts (30 %) and stearic acid in differing amounts (20 %, 30 % and 40 % stearic acid based on the amount of Eudragit L100-55). Afterwards, the dissolution profiles of the obtained pellets have been determined by HPLC measurement (HPLC apparatus: e.g. Merck LaChrom L7100, L7200, and L7400; HP1100). For this purpose, the pellets containing the different enteric coatings have been pre-treated for 2 hours in 1000 ml 0.05 M sodium acetate (pH 4.5), followed by filtering off of the pellets. The pellets were then transferred to 900 ml 0.1 Na₂HPO₄ (pH 6.8), where the solution containing the pellets has been stirred (paddle apparatus according to the requirements as described in the European Pharmacopoeia, chapter 2.9.3., Version 01/2008: 20903) with a paddle speed of 100 rpm (rotations per minute)). At the below indicated time points, samples (5 ml) are withdrawn, filtrated, and transferred to test-tubes each containing sodium hydroxide (40 %). The released API (esomeprazole) in % has been detected at different time points (0, 5, 15, 30, 45 minutes (min.)) at a wavelength of 303 nm (table II)

As can be seen from the following table (table II), the amount of stearic acid strongly influences the dissolution profile of the pellets. The more amount of stearic acid is present in the enteric coating, the more the release of the API is delayed. This becomes in particular clear at 5 minutes after having transferred the pellets to the solution having pH 6.8. However, there is no additional retardation effect, which can for instance be deduced from the fact that the API is largely released within a desired period of time (e.g. within 2 hours, or e.g. within 1.5 hours) after the transfer of the pellets in the solution having pH 6.8.

**Table II:**

| | **20 % Stearic acid** | **30 % Stearic acid** | **40 % Stearic acid** |
|---|---|---|---|
| **0 min.** | 0 % | 0 % | 0 % |
| **5 min.** | **34 %** | **30 %** | **12 %** |
| **15 min.** | 68 % | 66 % | 44 % |
| **30 min.** | 86 % | 87 % | 78 % |
| **45 min.** | 89 % | 91 % | 89 % |

### 2. Influence of the hydrophilic plasticizers propylene glycole and triethyl citrate on the dissolution profile

In order to show the influence of hydrophilic plasticizers (e.g. propylene glycole and triethyl citrate) on the dissolution profile of the enteric polymer, an API (esomeprazole), together with pharmaceutical acceptable excipients, has been applied onto non-pareilles (sugar cores). These non-pareilles in turn have been coated with various coatings containing Eudragit L100-55 and a separating agent (stearic acid) in constant amoumts (40 % based on the amount of Eudragit L100-55) and plasticizers in different amounts (0 %, 30 % and 55 % triethyl citrate or propylene glycole based on the amount of Eudragit L100-55). Afterwards, the dissolution profiles of the obtained pellets have been determined by HPLC measurement (HPLC apparatus: e.g. Merck LaChrom L7100, L7200, and L7400; HP1100). For this purpose, the pellets containing the different enteric coatings have been pre-treated for 2 hours in 1000 ml 0.05 M sodium acetate (pH 4.5), followed by filtering off of the pellets. The pellets were then transferred to 900 ml 0.1 Na₂HPO₄ (pH 6.8), where the solution containing the pellets has been stirred (paddle apparatus according to the requirements as described in the European Pharmacopoeia, 2.9.3., Version 01/2008: 20903) with a paddle speed of 100 rpm (rotations per minute)). At the below indicated time points, samples (5 ml) are withdrawn, filtrated, and transferred to test-tubes each containing sodium hydroxide (40 %). The released API (esomeprazole) in % has been detected at different time points (0, 5, 15, 30, 45 minutes (min.)) at a wavelength of 303 nm (tables III and IV).

As can be deduced from the following tables (tables III and IV), the amount of plasticizer strongly influences the dissolution profile of the pellets in that the addition of plasticizers leads to a reduction of the retardation time, wherein with increasing amounts of plasticizers added the dissolution of the API increases. This effect becomes in particular clear at 5 minutes after having started the disintegration test. Again it is noted that no additional retardation effect is present.

**Table III, plasticizer: Propylene glycole**

| | **40 % Stearic acid, 0 % Propylene glycole** | **40 % Stearic acid 30 % Propylene glycole** | **40 % Stearic acid 55 % Propylene glycole** |
|---|---|---|---|
| **0 min.** | 0 % | 0 % | 0 % |
| **5 min.** | 3 % | 12 % | 32 % |
| **15 min.** | 40 % | 44 % | 63 % |
| **30 min.** | 75 % | 78 % | 79 % |
| **45 min.** | 87 % | 89 % | 85 % |

**Table IV, plasticizer: Triethyl citrate**

| | **40 % Stearic acid, 0 % Triethyl citrate** | **40 % Stearic acid 30 % Triethyl citrate** | **40 % Stearic acid 55 % Triethyl citrate** |
|---|---|---|---|
| **0 min.** | 0 % | 0 % | 0 % |
| **5 min.** | **3 %** | **9 %** | **12 %** |
| **15 min.** | 40 % | 43 % | 52 % |
| **30 min.** | 75 % | 81 % | 82 % |
| **45 min.** | 87 % | 93 % | 90 % |

## Claims

1. An enteric coating comprising an enteric polymer or copolymer, a fatty acid and a hydrophilic plasticizer in an amount of equal to or more than 5 wt.-% and equal to or less than 60 wt.-%, based on the amount of the enteric polymer.

2. The enteric coating according to claim 1, wherein the enteric polymer is an anionic polymer or copolymer with methacrylic acid as a functional group.

3. The enteric coating according to claim 1 or 2, wherein the fatty acid contains at least 6 C-atoms.

4. The enteric coating according to any of the preceding claims, wherein the enteric coating contains the fatty acid in an amount of between 5.5 wt.-% and 200 wt.-% based on the amount of the enteric polymer.

5. The enteric coating according to any of the preceding claims, wherein the fatty acid does not provide an additional retardation effect.

6. The enteric coating according to any of the preceding claims, wherein the coating comprises between equal to or more than 20 wt.-% and equal to or less than 40 wt.-% of plasticizer, based on the amount of enteric polymer.

7. The enteric coating according to any of the preceding claims, wherein the hydrophilic plasticizer is selected from the group consisting of phthalic derivatives, dibutylphthalate, butylphthalylbutylglycolate, propylene glycol,triacetine, silicone oil, diethylphthalate, triethyl citrate, dibutyl sebacate, polyethylene glycol and propylene glycol.

8. A dosage form, comprising an enteric coating as defined in any one of claims 1 to 7.

9. The dosage form according to claim 8, wherein the dosage form represents a solid oral dosage form, preferably the dosage form is a tablet, preferably a multiple unit tablet, a microtablet or a capsule, which is coated with the enteric coating, or the dosage form contains crystals, granules, or pellets, which are coated with the enteric coating.

10. The use of a fatty acid, preferably a fatty acid as defined in claim 3, for the manufacture of an enteric coating.

11. The use according to claim 10, wherein the enteric coating is an enteric coating as defined in any one of claims 1 to 7.

12. An enteric coating composition, comprising a fatty acid,
wherein the composition comprises between equal to or more than 5 wt.-% and equal to or less than 60 wt.-%, preferably between equal to or more than 20 wt.-% and equal to or less than 40 wt.-% of plasticizer based on the amount of enteric polymer.

13. The enteric coating composition according to claim 12, wherein the composition contains an anionic polymer or copolymer as defined in claim 2.

14. The use of an enteric coating composition as defined claim 12 or 13 for the coating of solid oral dosage forms.

15. A process for the preparation of a solid oral dosage form,
wherein an enteric coating composition as defined in claim 12 or 13 is sprayed at least onto parts of a solid oral dosage form.
